# EUROPEAN PATENT SPECIFICATION

(11) **EP 1 255 553 B1**
(45) Date of publication and mention of the grant of the patent: **13.10.2010**
(21) Application number: 00948147.4
(22) Date of filing: 25.07.2000
(51) Int. Cl.: A61K 31/565, A61K 31/138, A61K 31/00, A61P 35/00

(54) **USE OF TRILOSTANE OR KETO-TRILOSTANE IN COMBINATION WITH AN ANTIOESTROGEN IN THE TREATMENT OF A HORMONE-DEPENDENT CONDITION.**
VERWENDUNG VON TRILOSTAN ODER KETO-TRILOSTAN IN KOMBINATION MIT EINEM ANTI-OESTROGEN ZUR BEHANDLUNG VON HORMON-ABHÄNGIGEN KRANKHEITEN.
UTILISATION DE TRILOSTANE OU DE KETO-TRILOSTANE EN COMBINAISON AVEC UN AGENT ANTIESTROGENIQUE DANS LE TRAITEMENT DE MALADIES DEPENDANTS DE HORMONES

(30) Priority: 18.01.2000 GB 0000984
(43) Date of publication of application: 13.11.2002
(73) Proprietor: Stegram Pharmaceuticals Limited, 14 Oakhurst Rise Carshalton Surrey SN5 4AG (GB)
(72) Inventor: Margetts, George, Billingshurst RH14 9TN (GB); Puddefoot, John, Upminster, Essex RM14 3DS (GB); Vinson, Gavin Paul, London N10 3BE (GB)
(74) Representative: Benson, John Everett
(86) International application number: PCT/GB2000/002858
(87) International publication number: WO 2001/053321

(56) References cited:
- EP-A- 0 154 476
- EP-A- 0 296 097
- IINO Y ET AL: "EFFECTS OF TRILOSTANE ON 7 12 DIMETHYLBENZ-ALPHA-ANTHRACENE-INDUCED RAT MAMMARY CANCERS AND BODY WEIGHT OF RATS IN RELATION TO ESTROGEN RECEPTORS" ONCOLOGY (BASEL), vol. 46, no. 5, 1989, pages 301-305, XP001032442 ISSN: 0030-2414
- GELDOF A A ET AL: "Inhibition of 3-beta-Hydroxysteroid-Dehydrogenase: An Approach for Prostate Cancer Treatment?" ANTICANCER RESEARCH, vol. 15, no. 4, 1995, pages 1349-1354, XP001032428 ISSN: 0250-7005
- ALIVIZATOS G. ET AL: "Update of hormonal treatment in cancer of the prostate." ANTI-CANCER DRUGS, (1993) 4/3 (301-309). , XP000651103
- INGLE J N ET AL: "EVALUATION OF TRILOSTANE PLUS HYDROCORTISONE IN WOMEN WITH METASTATIC BREAST CANCER AND PRIOR HORMONAL THERAPY EXPOSURE" AMERICAN JOURNAL OF CLINICAL ONCOLOGY, vol. 13, no. 2, 1990, pages 93-97, XP001032426 ISSN: 0277-3732
- RUBENS R D: "Treatment of metastatic breast cancer and its complications." CURRENT OPINION IN ONCOLOGY, (1991 DEC) 3 (6) 1029-34. REF: 32 , XP001032555
- HARMSEN JR. H.J. ET AL: "Endocrine therapy of breast cancer." EUROPEAN JOURNAL OF CANCER AND CLINICAL ONCOLOGY, (1988) 24/7 (1099-1116). , XP001032439
- HINDLEY A C ET AL: "A COMPARISON OF 2 DOSES OF TRILOSTANE COMBINED WITH DEXAMETHASONE IN METASTATIC BREAST CANCER" BRITISH JOURNAL OF CANCER, vol. 50, no. 2, 1984, page 252 XP001032449 ISSN: 0007-0920
- TUENI E ET AL: "ENDOCRINE EFFECTS OF TRILOSTANE IN-VITRO AND IN-VIVO STUDIES" EUROPEAN JOURNAL OF CANCER & CLINICAL ONCOLOGY, vol. 23, no. 10, 1987, pages 1461-1468, XP001032430 ISSN: 0277-5379
- COOMBES R.C. ET AL: "Trilostane therapy for advanced breast cancer." CANCER TREATMENT REPORTS, (1985) 69/4 (351-354). CODEN: CTRRDO, XP001032444
- WILKINSON J R ET AL: "RESPONSE OF NITROSOMETHYLUREA-INDUCED RAT MAMMARY TUMOR TO ENDOCRINE THERAPY AND COMPARISON WITH CLINICAL RESPONSE" CANCER RESEARCH, vol. 46, no. 9, 1986, pages 4862-4865, XP001032443 ISSN: 0008-5472
- WILLIAMS C J ET AL: "Multicentre cross over study of aminoglutethimide and trilostane in advanced postmenopausal breast cancer." BRITISH JOURNAL OF CANCER, vol. 68, no. 6, 1993, pages 1210-1215, XP001032429 ISSN: 0007-0920
- TOUEINI E.A. ET AL: "[Optimal hormonal combinations for therapy of advanced breast cancer. New therapeutical approach?]. UTILISATION OPTIMALE DES COMBINAISONS HORMONALES POUR LE TRAITEMENT DU CANCER DU SEIN AU STADE AVANCE. NOUVELLES PERSPECTIVES THERAPEUTIQUES?." LOUVAIN MEDICAL, (1986) 105/2 (111-120). CODEN: LOMEAL, XP001032525

## Description

The present invention relates to the use of (i) an antioestrogen and (ii) Trilostane or keto-trilostane in the treatment of hormone dependent conditions. Trilostane and related steroids have been found by the inventors to modulate specific isoforms of the oestrogen receptor(s) on human and animal cells. The effect of such modulation is to change the binding characteristics of ligands, in particular oestrogen, at the receptor site.

The selective modulation of steroid receptors in human and animal cells is the current basis for the hormonal treatment of various cancers including breast and prostate cancer in humans. The present invention relates to the potential use of the above compounds in combination with an antioestrogen to treat hormone-dependant disease, and in particular cancers.

Oestrogen is a vital hormone in the human and animal body and plays a crucial role in the development and maintenance of organ function. However, there are also well documented deleterious effects of oestrogen, such as that in some persons it promotes cancer, for example in the breast and uterus.

Oestrogen acts on cells in the body via nuclear proteins called oestrogen receptors (ER). The receptors not only bind with oestrogen, but can also change shape, form pair and link to oestrogen response elements (EREs) in certain genes. Attachment to EREs triggers formation of a transcription complex of co-activator proteins which in turn activates the gene(s). This induces a transcription enzyme (RNA polymerase) to transcribe messenger RNA, the template from which proteins are formed that induces the required change in the target cell.

ER also has an important relationship with the progesterone receptor (PgR) which has been identified in many tissues. There is considerable evidence that oestrogens induce the PgR gene through occupied ER binding to an ERE in the initiation region of the PgR gene. In breast cancers the response to endocrine therapy bears a strong relationship to the ER and PgR content of the cancer cells.

For many years it was believed that only one form of the oestrogen receptor existed, now called the alpha receptor (ERα). However, experiments have shown that various molecular forms of the oestrogen receptor exist (Marsigliante S, Puddefoot J et al, J. Steroid Biochem. & Mol. Biol., 1991, 39; 703-711 and Baker VA, Puddefoot J et al, Br. J. Cancer, 1992, 66; 1083-1089). After sucrose gradient centrifugation, ER obtained in the soluble fractions is recovered either as a large 8S complex or as a smaller 4S form. Furthermore, the 4S RT can be resolved into three components with isoelectric point (pI) values of 6.3, 6.6 and 6.8 while the 8S ER focuses as a single isoform at pI 6.1. Isoelectric focusing techniques, high performance liquid chromatography and DEAE-cellulose chromatography have also shown the presence of ER isoforms.

Breast cancers are highly heterogeneous, and show considerable variability in the profile of ER isoforms and their relationship to PgR levels. The ER isoform at pI 6.6 was present in 97% of ER positive breast cancers, the isoform at pI 6.1 in 83%, the pI 6.3 isoform in 39% of cancers and the pI 6.8 isoform in 33%, but there was evidence that PgR expression was greater if three or four isoforms were present. Only 12% of cancers contained the full complement of ER isoforms. The ER isoforms at pI 6.1 and 6.8 were found only in PgR positive cancers. This heterogeneity in the ER isoform profile and the correlation with PgR levels might explain the variability in response to endocrine therapy.

Recently, Jan-Ake Gustafsson and co-workers (Proc. Natl. Acad. Sci. U.S.A. (1996); 93; 5925-5930) have described a second form of oestrogen receptor, and this has been termed the beta receptor (ERβ) to distinguish it from the original oestrogen receptor, ERα. ERβ is highly homologous to ERα (J. Biol. Chem. (1997),272; 25832-25838) and activates expression of reporter genes containing oestrogen response elements in an oestrogen-dependent manner. PCR analyses indicate that ERβ is highly expressed in prostate and ovary and with moderate expression in many other tissues including breast, uterus and testis.

Although ERβ and ERα share a high degree of homology, ERβ has certain important characteristics that are different from ERα. Transcription activation of AP1 enhancer elements by oestrogen receptors requires ligand and the AP1 transcription factors Fos and Jun. In studies (Paech K et al, Science, 1997; 277: 1508-1510) in which a luciferase reporter gene under control of an AP1 element was co-transfected with ERα in HeLa cells, both the oestrogen agonists and antagonists examined were able to activate transcription. When ERβ was co-transfected instead of ERα, oestradiol (E₂) and diethylstilboestrol (DES) were unable to stimulate AP1-mediated transcription in the HeLa cells. Similar findings were reported in Ishikawa, MCF7 and MDA453 cells, although in these cell lines, E₂ and DES-liganded ERβ acted as antagonists at the AP1 site and inhibited raloxifene stimulation. Thus, when E₂ and DES bind to ERβ, they act as antagonists to oestrogen-responsive genes at the AP1 site.

A splice variant of ERβ was described in 1998 (Peterson et al, Endocrinology, 1998; 139: 1082-1092) and designated as ERβ 2, with re-designation of the original as ERβ 1. The affinity of ERb2 for oestrogen is 35-fold lower than that of ERβ 1 and the concentration of E₂ needed to produce half-maximal response with ERβ 2 was approximately 1,000-fold greater than that for ERβ 1.

Oestrogen receptor beta was first originated from a rat prostate cDNA library and its mRNA is prevalent in rat and human prostates. In the rat, ERβ levels are comparable to those in other highly expressing reproductive organs such as the ovary, endometrium and testis. By contrast, ERβ expression in the human prostate is low relative to expression in the testis. ERβ expression is also regulated by androgens since in animals its mRNA is markedly decreased within 24 hr after castration, and the expression is restored rapidly with testosterone replacement. Findings from prostate cell lines indicate that ERβ is the key mediator of oestrogen-mediated events in the prostate. Of considerable interest is the finding that ERβ knockout mice show signs of prostatic hyperplasia with aging, which suggests that ERβ may protect against abnormal growth (Krege et al, Proc. Natl. Acad Sci., USA 1998; 95: 15677-15682).

Trilostane, (4α,5α-17β)-4,5-epoxy-3,17-dihydroxyandrost-2-ene-2carbonitrile, has the formula 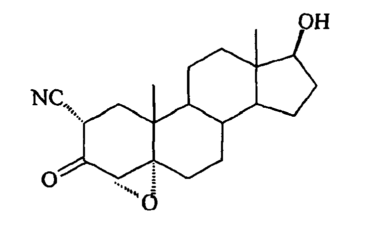 and is described in British Patent No. 1,123.770 and in U.S. Patent No. 3,296,295. These specifications describe the adrenocortical inhibiting properties of Trilostane and related compounds.

GB-B-2,130,588 relates to an improved method of manufacture for Trilostane and related compounds. This method allows the micronising of the compounds to particles having a mean equivalent sphere volume diameter of from 5 to 12mm, with at least 95% of the particles having a particle diameter of less than 50mm. The greater specificity of particle size improves the bio-availability of Trilostane and controls the amount of active metabolite formed, thus improving the clinical response and reducing variability.

Trilostane has been extensively studied as a treatment of advanced breast cancer. Several published studies confirm the efficacy of Trilostane with response rates of between 29% (Williams C.J.et al, Brit.J.Cancer (1993).68, 1210-1215) and 38% (Ingle J.N. et al, Am.J.Clin.Oncol., 1990, 13(2), 93-97).

Maruyama K et al (Biochem. Biophys. Res. Commun. 1998; 246: 142-147) reported that the presence of ERβ 2 may suppress ERβ 1 or ERα-mediated transcriptional activity in response to oestrogen. More recently, Hall JM, & McDonnell DP, (Endocrinology 1999 Dec;140(12):5566-78) determined that ERβ functions as a transdominant inhibitor of ERα transcriptional activity at subsaturating hormone levels and that ERβ decreases overall cellular sensitivity to estradiol. Additionally, they found that the partial agonist activity of tamoxifen manifest through ERα in some contexts was completely abolished upon coexpression of ERβ.

The anti-oestrogens, notably tamoxifen, have played an important role in the treatment of breast cancer. Tamoxifen has been in use for many years and has been shown to have a significant response rate in ER positive breast cancer. The finding of a decrease in contralateral breast cancer incidence following tamoxifen administration for adjuvant therapy led to the concept that the drug might play a role in breast cancer prevention. A recent report of a study conducted by the National Surgical Adjuvant Breast and Bowel Project (J. Natl. Cancer Inst. 1998, Sep 16;90(18):1371-88) showed tamoxifen reduced the risk of invasive breast cancer by 49% (two-sided P<.00001), with cumulative incidence through 69 months of follow-up of 43.4 versus 22.0 per 1000 women in the placebo and tamoxifen groups, respectively. The decreased risk occurred in women aged 49 years or younger (44%), 50-59 years (51%), and 60 years or older (55%); risk was also reduced in women with a history of breast lobular carcinoma in situ (56%) or atypical breast hyperplasia (86%) and in those with any category of predicted 5-year risk. Tamoxifen reduced the risk of noninvasive breast cancer by 50% (two-sided P<.002). Tamoxifen reduced the occurrence of oestrogen receptor-positive tumors by 69%, but no difference in the occurrence of oestrogen receptor-negative tumors was seen.

However, an important side-effect was noted in that the rate of endometrial cancer was increased in the tamoxifen group (risk ratio = 2.53; 95% confidence interval = 1.35-4.97); this increased risk occurred predominantly in women aged 50 years or older. All endometrial cancers in the tamoxifen group were stage I (localized disease) and no endometrial cancer deaths occurred in this group. The ability of tamoxifen to stimulate the endometrium has been recognised for some time, and the formation of endometrial cancers is a worrying and unwanted complication. The endometrial stimulation is believed to be the result of an agonist effect on the ERα receptors in the uterus. However, it may also be due to blocking of both ERα and ERβ receptors in the uterus by tamoxifen and other medicaments.

Another widely recognised complication of anti-oestrogen therapy, and especially tamoxifen, for breast cancer is the development of drug resistance. The nature and cause of tamoxifen resistance is unknown but it may be due to the blockade of both ERα and ERβ. Oestradiol binding to ERβ at the AP1 site causes down-regulation of transcription, which is equivalent to "switching off' the cell's activity. Tamoxifen, on the other hand, activates transcription of ERβ at the AP1 site and thereby effectively "switches on" the cell's activity. This may be the basis for the well recognised partial agonist action of tamoxifen and other similar compounds as well as their role as oestrogen antagonists.

In all previous work with Trilostane it was believed that the mode of action was by competitive inhibition of the 3β-hydroxysteroid dehydrogenase Δ 4,5 isomerase enzyme system. The action was thought to be due to androgen depletion and hydrocortisone was given with Trilostane to over-ride any feed-back mechanism. The compounds were shown to have no direct action on the then known oestrogen, androgen or progesterone receptors.

The inventors have, surprisingly, found that Trilostane and related compounds modulate principally the binding of ligand to the oestrogen receptor pI 6.3 isoform. Since ERβ corresponds to the pI 6.3 isoform in both molecular weight and positive immuno-reaction with a specific monoclonal antibody raised to ERβ receptor, the modulation of ligand binding to the pI 6.3 isoform by Trilostane and related compounds is therefore, by implication, modulation of the ERβ oestrogen receptor.

The inventors have further found that binding of competitive glands, such as the anti-oestrogen tamoxifen and raloxifene to the oestrogen receptor is enhanced in the presence of Trilostane and the major metabolite keto-trilostane. Thus improved treatment of hormone dependant disease with anti-oestrogens by combination with treatment with Trilostane or keto-trilostane is provided by the present invention.

Another unexpected finding underlying the present invention is that Trilostane acts by modulating oestrogen binding to the ER receptor specifically by increasing binding to ERβ and increasing the affinity of oestrogen binding and by inhibiting the stimulatory actions of oestrogen mediated by the ERα receptor. Thus Trilostane and related compounds will block the endometrial stimulation caused by oestrogen antagonists such as tamoxifen and raloxifene. Trilostane or keto-trilostane can therefore be used during treatment with oestrogen antagonists to reduce the unwanted side-effects of the oestrogen antagonists.

The novel action of Trilostane and related steroid compounds in selectively increasing oestradiol binding at the ERβ receptor and inhibiting ERα mediated activities has considerable significance for the treatment of certain hormone-dependent diseases including hormone dependant cancers, especially cancer of the breast, prostate and ovary. Therefore the present invention provides for the treatment of hormone-dependent diseases with anti-oestrogenic compounds in combination with treatment with Trilostane or keto-trilostane which will reduce or reverse the development of resistance to said anti-oestrogenic compound.

The finding of the present invention show that Trilostane and its related steroid compounds can be used in combination with antioestrogens such as tamoxifen or raloxifene, in the treatment of certain diseases, in particular cancers, since Trilostane appears to act on different oestrogen receptor isoforms and in different parts of the receptor isoforms to these agents.

Accordingly, the present invention provides: use of (i) an antioestrogen and (ii) trilostane or keto-trilostane in the manufacture of a medicament for the treatment of a hormone dependent condition by modulation of the oestrogen receptor β (Erβ) wherein the treatment comprises simultaneous administration of said trilostane or keto-trilostane and said antioestrogen;

A product containing (a) trilostane or keto-trilostane and (b) an antioestrogen for combined use, by simultaneous administration, in the treatment of a hormone dependant condition by modulation of the oestrogen receptor β (ERβ);

Use of trilostane or keto-trilostane in the manufacture of a medicament for the enhancement of the activity of an antioestrogen used for the treatment of a hormone dependent condition, wherein the treatment comprises simultaneous administration of said trilostane or keto-trilostane and said antioestrogen.

Use of trilostane or keto-trilostane in the manufacture of a medicament for the reduction of unwanted side-effects of an antioestrogen used for the treatment of a hormone-dependent condition;

Use of a trilstane or keto-trilostane in the manufacture of a medicament for reducing or reversing the development of resistance to an antioestrogen used for the treatment of a hormone dependent condition;

Use of trilostane or keto-trilostane in the manufacture of a medicament for use in the treatment of a hormone-dependent condition by modulation of the oestrogen receptor β by simultaneous administration with an antioestrogen; and

Use of an antioestrogen in the manufacture of a medicament for use in the treatment of a hormone-dependent condition by modulation of the oestrogen receptor β by simultaneous administration with trilostane or keto-trilostane.

### BRIEF DESCRIPTION OF THE DRAWINGS

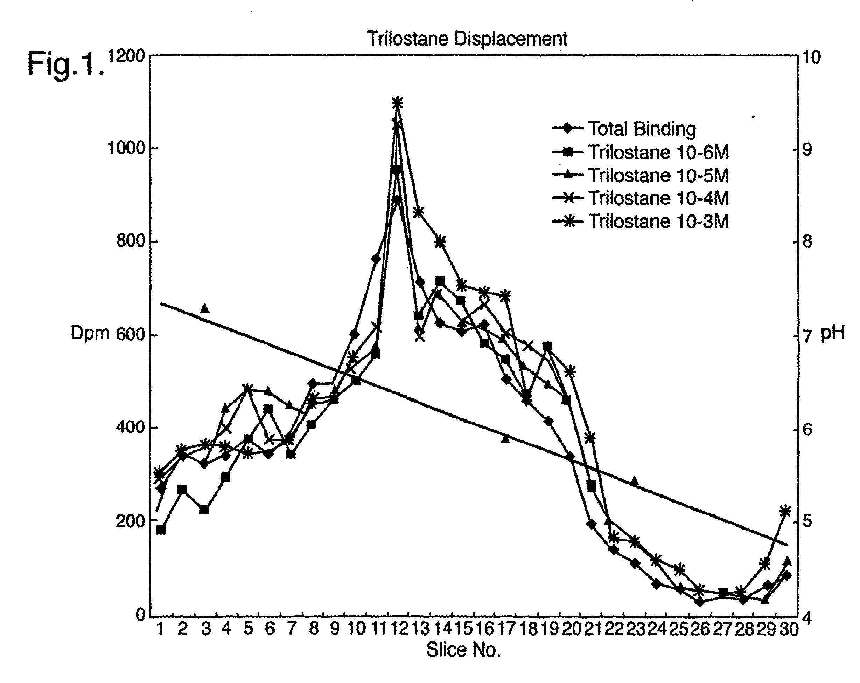

Figure 1. Graph showing the action of Trilostane at different concentrations on tritiated oestradiol (5nM) binding to the pI 6.3 isoform (Erβ) of the oestrogen receptor in rat uterus. The graph shows tritiated oestradiol (in DPM) recovered in sequestered slices from IEF gel (see text).

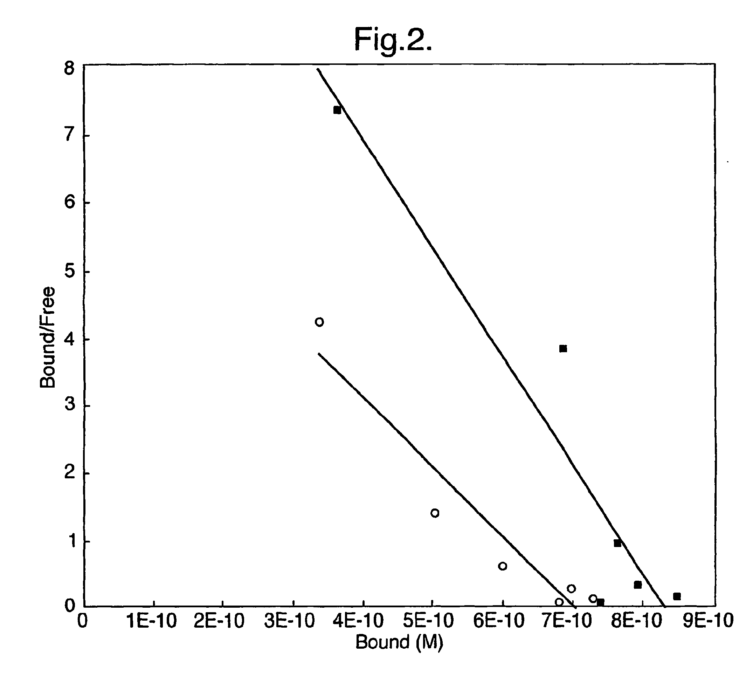

Figure 2. Scatchard plots for the tritiated oestradiol binding to rat uterine oestrogen receptors in the absence (open symbols) and presence (closed symbols) of Trilostane at 10micromolar. Presence of Trilostane increases the apparent affinity of oestrogen binding (given by the slope of the regression lines) and the concentration of total receptor (given by the X axis intercept).

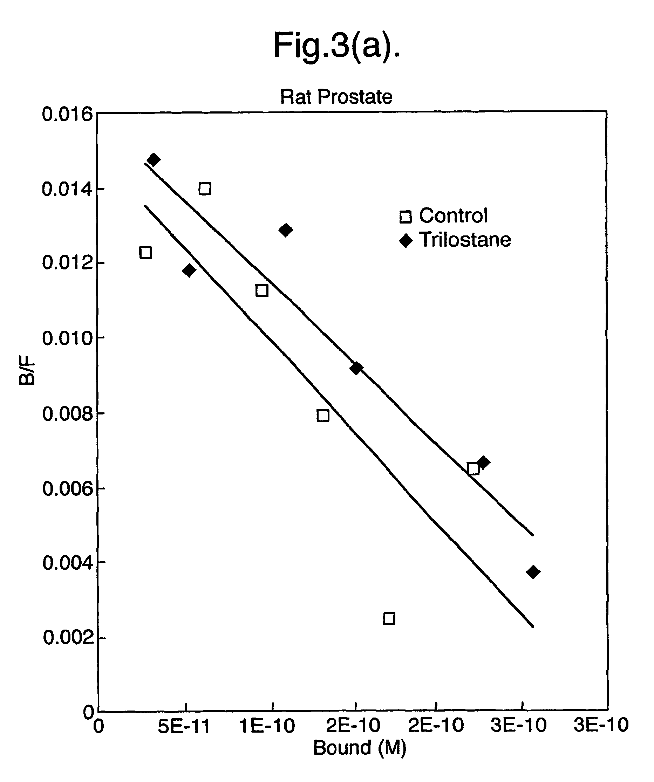

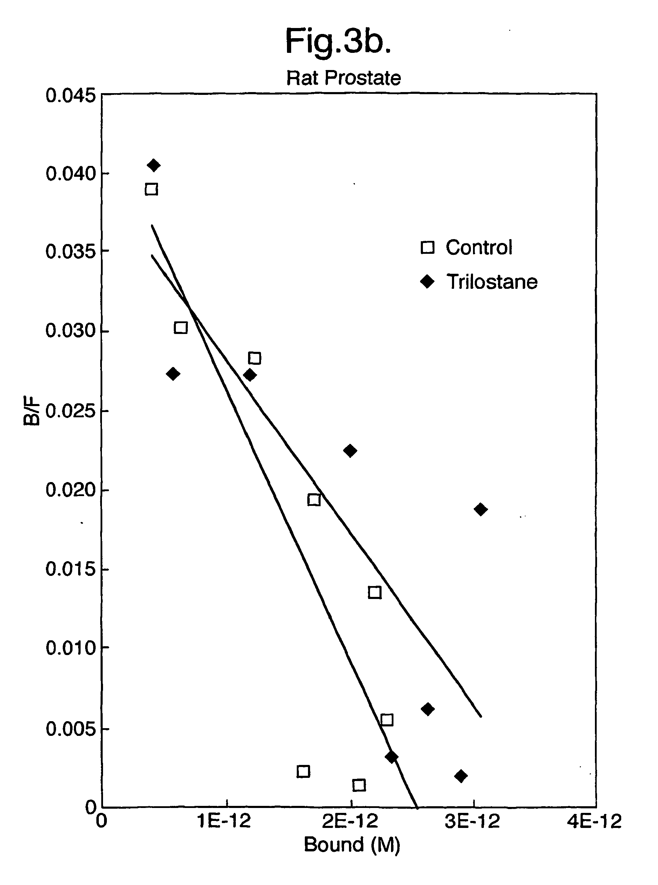

Figure 3. Scatchard plots for the tritiated oestradiol binding to rat prostate oestrogen receptors in the absence (open symbols) and presence (closed symbols) of Trilostane at 10mM. Presence of Trilostane increases the concentration of total receptor (given by the x axis intercept).

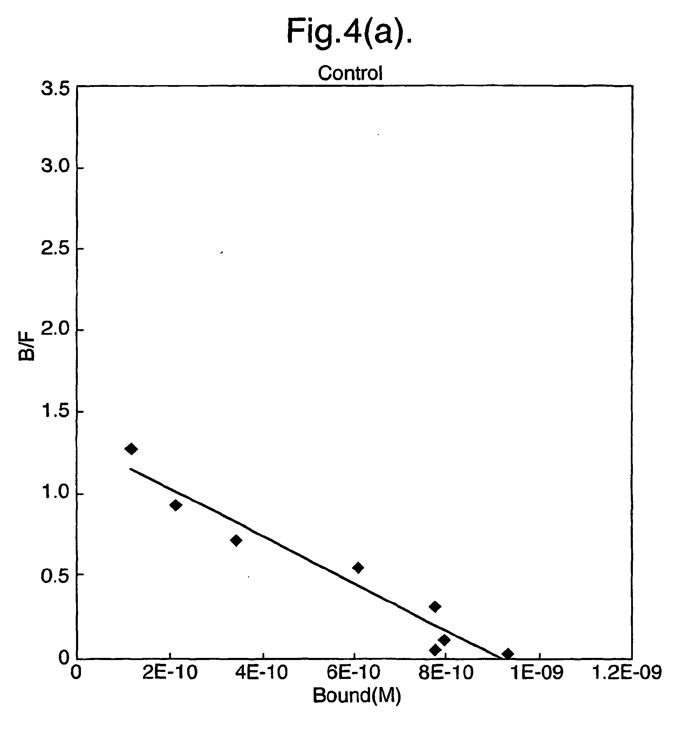

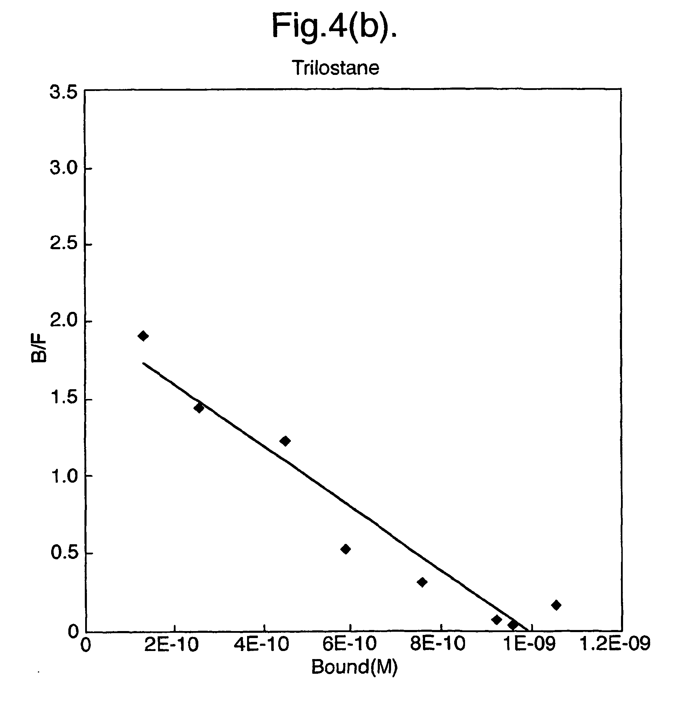

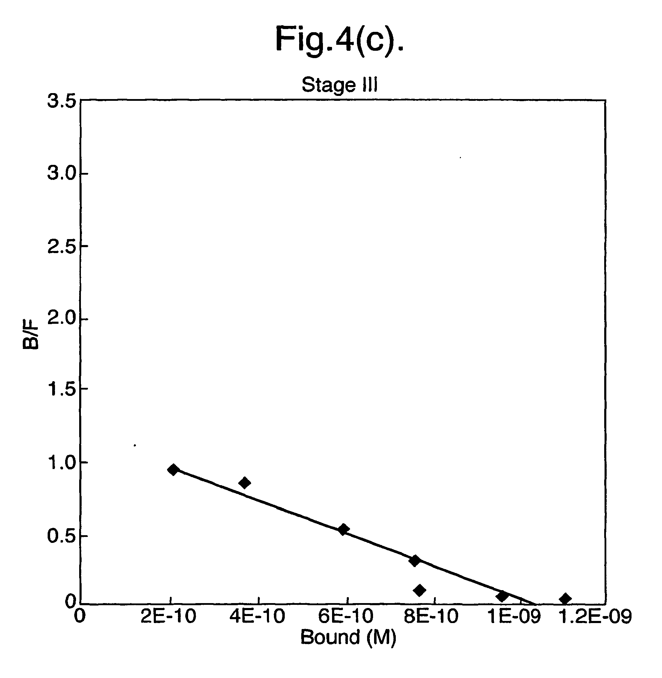

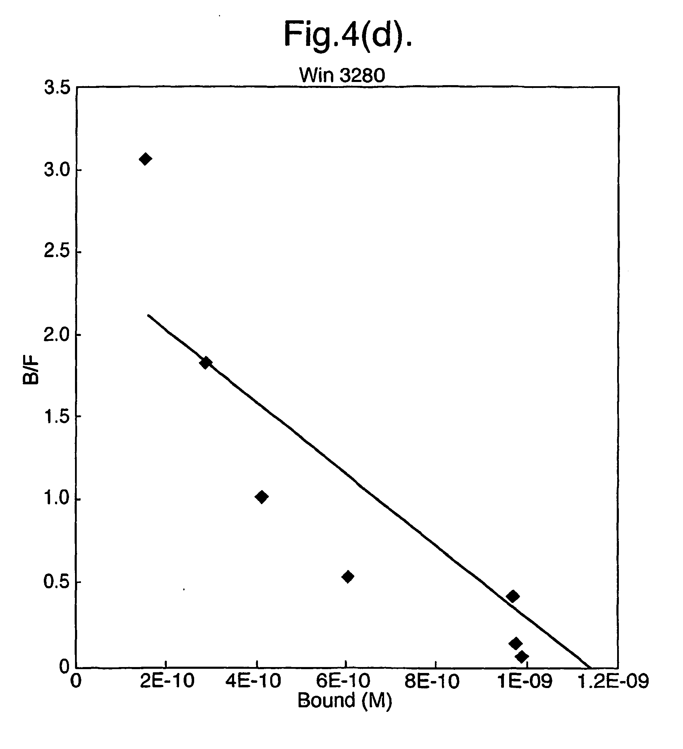

Figure 4. Scatchard plots for the tritiated oestradiol binding to rat uterus oestrogen receptors a) for control i.e. no additional compounds, and in the presence of (b) Trilostane, (c) (4α,5α-17β)-4,5-epoxyandrost-2-eno[iso-2,3-D]-azol-17-ol [Stage III compound] and (d) keto-trilostane. Presence of either Trilostane or keto-trilostane increases the apparent affinity of oestrogen binding (given by the slope of the regression lines), but no change in binding affinity occurs with the stage III compound.

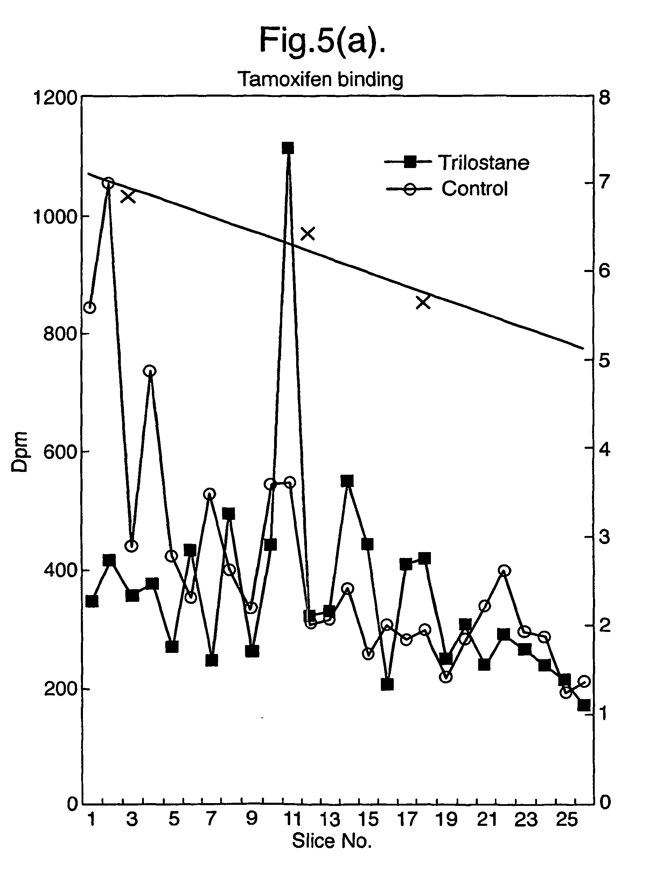

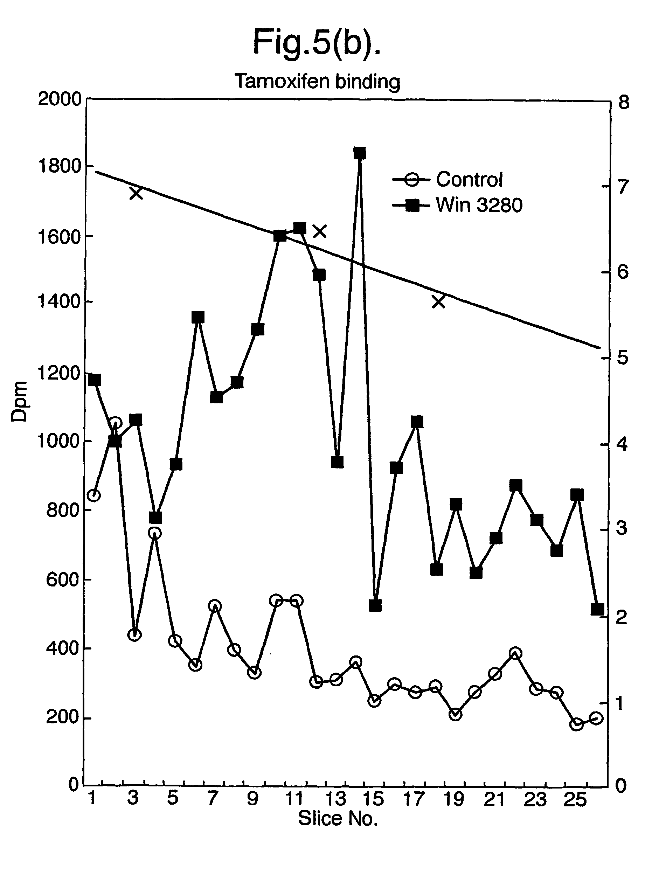

Figure 5. Effect of a) Trilostane and b) keto-trilostane on tritiated tamoxifen binding to oestrogen receptor isoforms in rat uterus. The graphs show increased tritiated tamoxifen binding to oestrogen receptor isoforms in the presence of either Trilostane (10 micromolar) or keto-trilostane (10 micromolar).

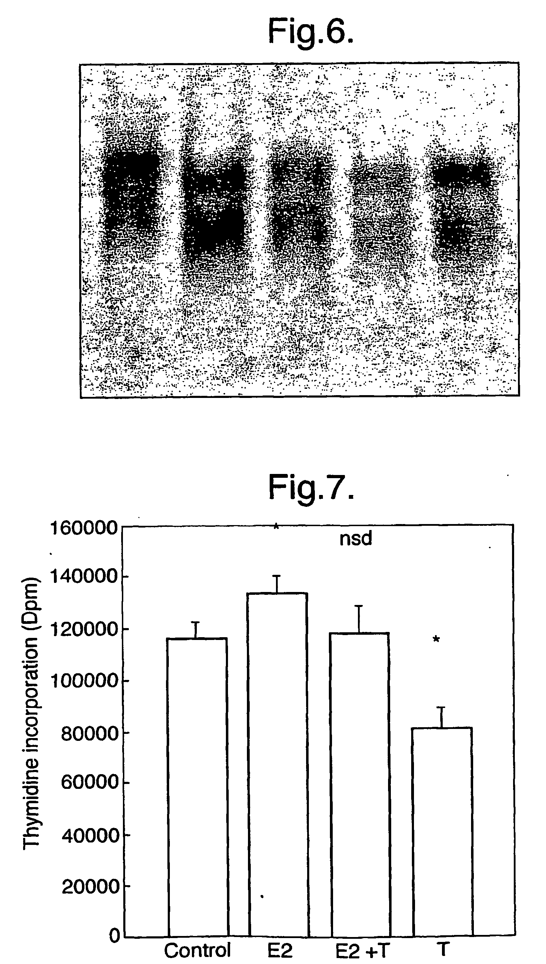

Figure 6. Electrophoretic mobility shift assay showing characteristic double band shift resulting from binding of oestrogen receptor present in MCF-7 breast cancer cell extracts to radiolabelled oestrogen response element run on 6 % native polyacrylamide gel. Serum-starved MCF-7 cells were treated for 24 hours with the following treatments which were replenished 4 hours prior to harvesting of cells for extract preparation.

Lane 1 = serum-free medium alone
Lane 2 = serum-free medium + estradiol (10⁻⁸M)
Lane 3 = serum-free medium + Trilostane (10⁻⁵M)
Lane 4 = serum-fiee medium + keto-trilostane (10⁻⁵M)
Lane 5 = serum-free medium + Tamoxifen (10⁻⁶M)

Figure 7. Thymidine labeling of MCF-7 cells incubated for 24 hours in the presence and absence of Oestradiol (10⁻⁸ M) and Trilostane (10µM). Cells grown in the presence of oestradiol (E2) show a 15% increase in thymidine labeling (p<0.01) when compared to control, which is completely blocked by the presence ofTrilostane (E2+T). In addition, Trilostane alone (T) inhibits thymidine uptake of MCF-7 cells by 31% compared with control (p<0.01).

### DETAILED DESCRIPTION OF THE INVENTION

Trilostane and keto-trilostane as shown in formula (I) may be used in the present invention.

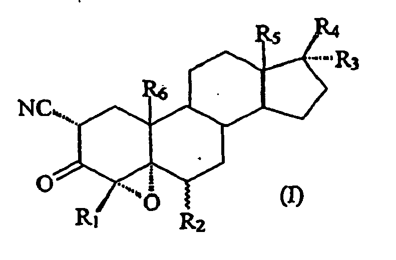

Trilostane: (R₁, R₂ and R₃ are hydrogen, R₄ is hydroxy and R₅ and R₆ are methyl), keto-Trilostane: (R₁ and R₂ are hydrogen, R₃ and R₄ together are oxo and R₅ and R₆ are methyl).

The compound of Trilostane or keto-trilostane may be used with an antioestrogen in the manufacture of a medicament for the treatment of a hormone dependant condition by modulation of the oestrogen receptor β, wherein the treatment comprises simultaneous administration of said trilostane or keto-trilostane and said antioestrogen.

In particular, such compounds may be used when the hormone dependant condition is an oestrogen dependant condition. In addition, the compounds may be used when the hormone dependant condition is regulated by other hormones such as androgens or progesterone.

Notably conditions which may be treated include, but are not restricted to, oestrogen dependant cancers, for example breast cancer, prostate cancer, lung cancer, gastro-intestinal cancer, ovarian cancer, endometrial cancer, benign prostatic hypertrophy and endometrial hyperplasia.

Trilostane and keto-trilostane are preferably used in particulate form. In particular, the compounds desirably consist of particles having a mean particle diameter of about 12µm or less and 80, 85, 90, 95% or more, preferably 98% or more, 99% or more or 99.5% or more of the particles have a particle diameter of less than about 50µm, preferably less than 40µm, less than 30 µm or less than 20 µm e.g. from 0.1 µm to 10, 20, 30, 40 or 50 µm, 1 µm to

10, 20, 30, 40 or 50 µm or 10 to 20, 30, 40 or 50 µm. The particles preferably have a mean particle diameter of about 5µm to about 12µm or less than about 5µm, for example from 0.1 to 5µm or 1 to 5 µm. It is further preferred that the cumulative percentage oversize versus size characteristic curve of the compound of formula (I) exhibits a standard deviation of about 1.5 to 2.5µm, preferably about 1.75 to 2.25µm, more preferably about 2µm, e.g. 1.9 to 2.1 µm.

The treatment is given in the form of a medicament, which is preferably comprises a unit dosage of from about 50mg to 250mg, for example from 50mg to 100mg, from 100mg to 200mg or from 200mg to 250mg, of the compound of the present invention. The medicament may be administered by intravenous, intramuscular or subcutaneous route or as an ointment, cream or lotion. The preferred route is oral, either as a tablet, a capsule or a suspension.

The treatment is used in combination with further treatment with one or more antioestrogen compounds such as, but not restricted to, tamoxifen and raloxifene.

The treatment and the further treatment is carried out simultaneously.

Trilostane or keto-trilostane may also be used in the manufacture of a medicament for the enhancement of the activity of an antioestrogen used for the treatment of a hormone dependant condition.

The treatment and the further treatment may be carried out simultaneously, separately or sequentially, and in either order if separate or sequential. The treatment and further treatment may be given in the form of a single combined medicament, which preferably comprises a unit dosage of said further compound in an amount known in the art to be effective in the treatment of said hormone dependant condition, and a unit dosage of a compound of the present invention in an amount as described above. The medicament may be administered by a mode as described above. Alternatively, the two treatments may be given separately or sequentially, e.g. as two different medicaments administered at the same site or at different sites, by the same mode of administration or by different modes of administration.

Trilostane or keto-trilostane may further be used in the manufacture of a medicament for the reduction of unwanted side-effects of an antioestrogen used for the treatment of a hormone dependent condition. The treatment and the further treatment may be carried out simultaneously, separately or sequentially, and in either order if separate or sequential. The treatment and further treatment may be given in the form of a single combined medicament, which preferably comprises a unit dosage of said further compound in an amount known in the art to be effective in the treatment of said hormone dependant condition, and a unit dosage of a compound of the present invention in an amount as described above. The medicament may be administered by a mode as described above. Alternatively, the two treatments may be given separately or sequentially, e.g. as two different medicaments administered at the same site or at different sites, by the same mode of administration or by different modes of administration.

Trilostane or keto-trilostane may also be used in the manufacture of a medicament for reducing or reversing the development of resistance to an antioestrogen used for the treatment of a hormone dependant condition. The treatment and the further treatment may be carried out simultaneously, separately or sequentially, and in either order if separate or sequential. The treatment and further treatment may be given in the form of a single combined medicament, which preferably comprises a unit dosage of said further compound in an amount known in the art to be effective in the treatment of said hormone dependant condition, and a unit dosage of a compound of the present invention in an amount as described above. The medicament may be administered by a mode as described above. Alternatively, the two treatments may be given separately or sequentially, e.g. as two different medicaments administered at the same site or at different sites, by the same mode of administration or by different modes of administration.

In the present invention it has been shown that Trilostane and related compounds have a specific action on some oestrogen receptor isoforms and thereby modulate the receptor and the binding of oestrogen to the receptor(s). The present invention therefore provides for a new role for Trilostane or keto-trilostane and allows for more specific and better-targeted therapy of hormone-dependant cancers and for the treatment of other diseases in which oestrogen receptors play a role in the aetiology or progression of that disease.

### EXAMPLES

Using tissue, snap frozen at time of excision, cytosols were prepared by dismembration using a Braun II Mikrodismembrator. Frozen tissue was minced and pulverised. The resultant powder was resuspended in 1-mm phosphate buffer containing 10% (v/v) glycerol, 1.5mm EDTA, 10mM monothioglycerol, 20mM Sodium molybdate, aprotinin (1ug/ml) and STI (1ug/ml) and phenyl methyl sulfonyl fluoride (30ug/ml) at pH 7.4 by stirring for 15 minutes at 4°C. This suspension was centrifuged at 100,000g for 1 hour at 4°C and the supernantant was used for competition analysis.

An aliquot of the supernatant was incubated with 3H oestradiol (5nM final concentration) in the presence or absence of either Trilostane at 10uM final concentration or Tamoxifen at 10⁻⁷M for 18 hours at 4°C. In additional experiments an aliquot of the supernatant was incubated with tritiated tamoxifen (10⁻⁶M) in the presence or absence of Trilostane (10⁻⁵M) or keto-trilostane (10⁻⁵M) for 18 hours at 4°C.

After overnight incubation free steroid was separated from bound by incubating with dextran coated charcoal (0.25% w/v charcoal, 0.025% w/v Dextran T70 in 10mM Tris 1.5mM EDTA at pH 7.4) for 10 mins at 4°C. The DCC was pelleted by centrifugation (10,000g for 10 mins) and aliquots of the supernatant were used for IEF separation.

For Scatchard analysis duplicate aliquots of the supernatants were incubated with increasing concentrations of 3H oestradiol in the presence and absence of a 100 fold excess of diethylstilboestrol. All tubes were then incubated in the presence or absence of Trilostane (final concentration 10uM). After overnight incubation at 4°C assay tubes were incubated with DCC as above to remove free steroid and aliquots of the resultant supernatant were incubated with toluene based scintillant and counted on a Scintillation Counter. Data obtained was analysed according to the method of Scatchard.

Isoelectric focusing (IEF) gels were cast in slabs of size 125 x 260 mm and separation was conducted along either the short side (short run) or the long side (long run) of the gel. Polyacrylamide gels (2 mm thick), containing 20% (v/v) glycerol and with high porosity (T=5%, C=3%) were used. A pH gradient of 5-10 was achieved using 1% (v/v) LKB ampholine 3.5-10 and 1.5% (w/v) LKB ampholine 5-8. Gels were photopolymerised at room temperature by means of a TR 26 polymerisation light, using riboflavin (0.004%) v/v) for at least 8 h. IEF was performed in a cold room and the temperature of the cooling water was kept constant at 4°C using an LKB Multiphor II system with its chamber filled with 1M NaOH to minimise CO2 absorption from the air. Electrode solutions of 1M NaOH (cathode) and 1M H2SO4 (anode) were used. Gels were pre-focused for 40 min at 20 mA/20W/1200 V (short run).

After DCC extraction, aliquots (270ul) of the radioactive supernatant (3 mg protein/ml) derived from SSD assay were loaded near the cathode. The runs were carried out for 4 h using a 3000xi CC power supply at 2500 V/20 mA/20 W, constant power (long run) and at 1200 V/20mA/20 W, constant power, for 1.5 h (short run). A mixture of nine natural proteins (Biorad) was used for pH calibration. After the run the gels were cut into 2.5 mm slices and each slice was incubated with 5ml scintillation cocktail for 24h at room temperature and radioactivity assayed.

Electro-mobility shift assay (EMSA) was performed on MCF-7 cells cultured in serum-free medium for 5 days with two medium replacements to deplete the cells of endogenous oestrogen. These serum-starved cells were then treated for 24 hours with either serum-free medium alone (control) or with serum-free medium containing b-estradiol (10⁻⁸M), Trilostane (10⁻⁵M), WIN 3280 (10⁻⁵M), or Tamoxifen (10⁻⁶M). 4 hours before harvesting this medium was replaced with fresh serum-free medium containing the corresponding addition, as above. Cells were then collected in phosphate-buffered saline (PBS) using a rubber policeman. Cell suspensions were centrifuged for 5 min at 4°C at 1000 X *g* then re-suspended in an equivalent volume of fresh ice-cold PBS and the centrifugation repeated. PBS was removed and cell pellets were re-suspended in extraction buffer (20 mM Hepes pH 7.8, 450 mM NaCl, 0.4 mM EDTA, 0.5 mM DTT, 25 % glycerol, 0.5 mM PMSF; Schreiber et al., Nucleic Acids Res., 1989, 17: 6419). Samples were subjected to three freeze-thaw cycles using a dry ice-ethanol bath and 37°C water bath and then centrifuged for 10 min at 4°C at 10 000 X g. Cell extracts were transferred to fresh microtubes and stored at -80°C prior to use.

Oligonucleotides representing an oestrogen response element sequence (ERE) were hybridised by heating to 80°C and allowing to cool to room temperature. The resultant double-stranded ERE was radiolabelled by end-labelling using Klenow Fragment and [a-³²P] dCTP (Amersham Pharmacia Biotech). Cell extracts were incubated for 30 min at room temperature in binding buffer (40 mM HEPES pH 7.4, 100 mM KCl, 2mM 2-mercaptoethanol, 20 % glycerol) containing 0.5 % bovine serum albumin and 50 mg poly[d(I-C)-d(I-C)]. After adding radiolabelled ERE (1 ng) extracts were incubated for a further 30 min at room temperature and then at 4°C for 30 min. 8 ml of cell extract was used per reaction. Samples were electrophoresed on a 6 % polyacrylamide native gel (pre-run for 30 min at 100 V) for 1 hour at 250 V at 4°C. After fixing the gel in 10 % acetic acid:30 % methanol for 15 min, gels were exposed to X-ray film overnight at -80°C with an intensifying screen.

The effect of Trilostane on transcriptional activity in the MCF-7 breast cancer cell line was determined using the oestrogen reporterplasmid xTG. The reporter plasmid xTG was designed so that oestradiol (E2) binding to native oestrogen receptor present within a cell would result in an accumulation of green fluorescent protein (GFP). XTG consists of a oestrogen response element. Upstream of a simple TATA promoter which together drive transcription of the GFP gene from the jellyfish *Aequorea victoria.*
The xERE sequence is made up of the following - GGTCA CAG TGACC
TTGATTCAAAGTTAATGTAACCTC (SEQUENCE ID NO: 1)

The GFP gene - from GFPuv was supplied by Clontech (Clontech Laboratories, Inc., 1020 East Meadow Circle, Palo Alto, CA 94303-4230, USA)

MCF-7 cells were plated out in multiwell dishes and grown for 24 hours in serum free medium (MEM). Cells were then incubated in serum free medium or serum free medium containing oestradiol alone (10⁻⁸ M), Trilostane alone (10⁻⁵M) or oestradiol and Trilostane together. Radiolabelled thymidine was added to each well (50mCi/ml) and cells were then cultured for a further 24 hours. At the end of this period the medium was aspirated and the cultured cells were rinsed 3-times with cold buffer solution(50mm Tris-HCl, pH 7.4). The cells were then dissolved in 1ml of 0.1N NaOH and 0.5ml of this solution was mixed with 2.5ml of Scintillation cocktail and the intracellular radioactivity of ³H was measured with a liquid scintillation counter.

In the IEF experiments described above Trilostane (10 micromolar) modulated the binding of oestrogen onto all the oestrogen receptor isoforms but principally onto the pI 6.3 isoform. By contrast tamoxifen (1 micromolar) displaced oestrogen from all the oestrogen receptor isofroms but principally the pI 6.1 isoform.

In experiments studying the effect of Trilostane on oestradiol binding kinetics in rat uterus we observed an altered affinity of receptors to steroid in that the kD changed from a lower affinity to a higher affinity while in rat prostate we observed an increase in receptor concentration.

Scatchard analysis performed in the presence of the major metabolite, keto-trilostane, also altered the kD to a higher affinity. However the presence of the stage III compound had no effect.

These data reflect the ability of Trilostane and related compounds to modulate certain functions and characteristics of the oestrogen receptor, such as the ability to modulate ligand binding to individual isoforms, receptor protein conformation, receptor dimerisation and binding co-operativity. These results are a novel and surprising finding for Trilostane and related compounds.

The pI 6.3 isoform of the oestrogen receptor was identified as the product of the ERβ gene on the basis of its molecular weight and positive immune reaction with a specific monoclonal antibody raised to the ERβ receptor. The pI 6.6 isoform corresponds with the ERα gene product.

Figure 6 shows the detection of bands which are characteristic of the formation of complexes between oestrogen receptor (ER) present in the MCF-7 cell extracts and the radiolabelled ERE. These complexes could be competed out by co-incubation with a 50-fold molar excess of unlabelled ERE but not by 50-fold excess of a probe representing the AP1 gene sequence, thus demonstrating the specificity of the ER-ERE interaction in this system.

In the absence of ligand (lane 1) ER-ERE complexes are apparent. However, extracts from cells treated with radio-labelled estradiol (lane 2) showed an increased level of ER-ERE complex formation (110 % of control density corrected for protein concentration in the extract. Treatment with Trilostane (lane 3) reduced complex formation to 96 % of control levels (lane 1), while keto-trilostane (lane 4) dramatically reduced ER-ERE complex formation to 68 % of control. Tamoxifen also reduced complex formation (lane 5) (93 % of control).

These data suggest that both Trilostane and keto-trilostane are able to modulate the conformation of the oestrogen receptor in such a way as to reduce the strength of interaction between this receptor and its response element. This clearly has profound implications with respect to the ability of oestrogen to activate specific genes in breast cancer cells. These compounds would be expected, therefore, to reduce the level of transcription of key estrogen-responsive genes in these cells.

Experiments using the cell line MCF-7 transiently transfected with xTG for three days, E2 only showed GFP accumulation whereas E2 plus Trilostane showed blue-green auto fluorescence from the transfection reagent only. This suggests that while E2 enhances production of GFP the antiestrogens tamoxifen and raloxifene reduced E2-driven transactivation. E2 was used at a concentration of 10-8M while all other compounds were used at 10-6M. All ligands were added to cell media during transfection. Under the same conditions Trilostane also reduced transcription of GFP.

Cells grown in the presence of oestradiol (E2) show a 15% increase in thymidine labeling (p<0.01) when compared to control, which is completely blocked by the presence of Trilostan (E2+T). In addition, Trilostane alone inhibits thymidine uptake of MCF-7 cells by 31% compared with control (p<0.01).

## Claims

1. Use of (i) antioestrogen and (ii) trilostane or keto-trilostane in the manufacture of a medicament for the treatment of a hormone dependent condition by modulation of the oestrogen receptor β, wherein the treatment comprises simultaneous administration of said trilostane or keto-trilostane and said antioestrogen.

2. Use of trilostane or keto-trilostane in the manufacture of a medicament for the enhancement of the activity of an antioestrogen used for the treatment of a hormone dependent condition, wherein the treatment comprises simultaneous administration of said trilostane or keto-trilostane and said antioestrogen.

3. Use of trilostane or keto-trilostane in the manufacture of a medicament for the reduction of unwanted side-effects of an antioestrogen used for the treatment of a hormone dependent condition.

4. Use of trilostane or keto-trilostane in the manufacture of a medicament for reducing or reversing the development of resistance to an antioestrogen used for the treatment of a hormone dependent condition.

5. Use of trilostane or keto-trilostane in the manufacture of a medicament for use in the treatment of a hormone dependent condition by modulation of the oestrogen receptor β by simultaneous administration with an antioestrogen.

6. Use of an antioestrogen in the manufacture of a medicament for use in the treatment of a hormone dependent condition by modulation of the oestrogen receptor β by simultaneous administration with trilostane or keto-trilostane.

7. Use according to any one of claims 1 to 6, wherein the hormone dependent condition is an oestrogen dependent condition.

8. Use according to any one of the preceding claims, wherein the hormone dependent condition is a hormone dependent cancer.

9. Use according to any one of the preceding claims, wherein the hormone dependent condition is an oestrogen dependent cancer.

10. Use according to any one of the preceding claims, wherein the hormone dependent condition is breast cancer, prostate cancer, lung cancer, gastro-intestinal cancer, ovarian cancer, endometrial cancer, benign prostatic hypertrophy, endometrial hyperplasia or endometrial hypertrophy.

11. Use according to any one of the preceding claims, wherein the trilostane or keto-trilostane is in particulate form.

12. Use according to claim 11, wherein the trilostane or keto-trilostane is in the form of particles having a mean particle diameter 0 to 12µm and 95% or more of the particles have a particle diameter of from 0 to 50µm.

13. Use according to claim 12, wherein the particles have a mean particle diameter of from 5 to 12µm.

14. Use according to claim 12, wherein the particles have a mean particle diameter of less than 5µm.

15. Use according to any one of the preceding claims, wherein the unit dosage of trilostane or keto-trilostane is from 50 mg to 250 mg.

16. Use according to any one of the preceding claims, wherein the antioestrogen is tamoxifen or raloxifene.

17. A product containing (a) trilostane or keto-trilostane and (b) an antioestrogen for combined use, by simultaneous administration, in the treatment of a hormone dependent condition by modulation of the oestrogen receptor β.

## Patentansprüche

1. Verwendung von (i) einem Antiöstrogen und (ii) Trilostan oder Ketotrilostan bei der Herstellung eines Arzneimittels zur Behandlung eines hormonabhängigen Zustands durch Modulation des Östrogenrezeptors β, wobei die Behandlung die gleichzeitige Verabreichung des Trilostans oder Ketotrilostans und des Antiöstrogens umfasst.

2. Verwendung von Trilostan oder Ketotrilostan bei der Herstellung eines Arzneimittels zur Verstärkung der Aktivität eines Antiöstrogens, das zur Behandlung eines hormonabhängigen Zustands verwendet wird, wobei die Behandlung die gleichzeitige Verabreichung des Trilostans oder Ketotrilostans und des Antiöstrogens umfasst.

3. Verwendung von Trilostan oder Ketotrilostan bei der Herstellung eines Arzneimittels zur Verringerung von unerwünschten Nebenwirkungen eines Antiöstrogens, das zur Behandlung eines hormonabhängigen Zustands verwendet wird.

4. Verwendung von Trilostan oder Ketotrilostan bei der Herstellung eines Arzneimittels zur Verringerung oder Umkehr der Entwicklung einer Resistenz gegen ein Antiöstrogen, das zur Behandlung eines hormonabhängigen Zustands verwendet wird.

5. Verwendung von Trilostan oder Ketotrilostan bei der Herstellung eines Arzneimittels zur Verwendung bei der Behandlung eines hormonabhängigen Zustands durch Modulation des Östrogenrezeptors β durch gleichzeitige Verabreichung mit einem Antiöstrogen.

6. Verwendung eines Antiöstrogens bei der Herstellung eines Arzneimittels zur Verwendung bei der Behandlung eines hormonabhängigen Zustands durch Modulation des Östrogenrezeptors β durch gleichzeitige Verabreichung mit Trilostan oder Ketotrilostan.

7. Verwendung nach einem der Ansprüche 1 bis 6, wobei es sich beim hormonabhängigen Zustand um einen östrogenabhängigen Zustand handelt.

8. Verwendung nach einem der vorstehenden Ansprüche, wobei es sich beim hormonabhängigen Zustand um einen hormonabhängigen Krebs handelt.

9. Verwendung nach einem der vorstehenden Ansprüche, wobei es sich beim hormonabhängigen Zustand um einen östrogenabhängigen Krebs handelt.

10. Verwendung nach einem der vorstehenden Ansprüche, wobei es sich beim hormonabhängigen Zustand um Brustkrebs, Prostatakrebs, Lungenkrebs, gastrointestinalem Krebs, Ovarialkrebs, Endometriumkrebs, gutartige Prostata Hypertrophie, Endometriumhyperplasie oder Endometriumhypertrophie handelt.

11. Verwendung nach einem der vorstehenden Ansprüche, wobei das Trilostan oder Ketotrilostan in teilchenförmiger Form vorliegt.

12. Verwendung nach Anspruch 11, wobei das Trilostan oder Ketotrilostan in Form von Teilchen mit einem durchschnittlichen Teilchendurchmesser von 0 bis 12 µm vorliegt und 95 % oder mehr der Teilchen einen Teilchendurchmesser von 0 bis 50 µm aufweisen.

13. Verwendung nach Anspruch 12, wobei die Teilchen einen durchschnittlichen Teilchendurchmesser von 5 bis 12 µm aufweisen.

14. Verwendung nach Anspruch 12, wobei die Teilchen einen durchschnittlichen Teilchendurchmesser von weniger als 5 µm aufweisen.

15. Verwendung nach einem der vorstehenden Ansprüche, wobei die Dosiseinheit von Trilostan oder Ketotrilostan 50 mg bis 250 mg beträgt.

16. Verwendung nach einem der vorstehenden Ansprüche, wobei es sich beim Antiöstrogen um Tamoxifen oder Raloxifen handelt.

17. Produkt, enthaltend (a) Trilostan oder Ketotrilostan und (b) ein Antiöstrogen zur kombinierten Verwendung durch gleichzeitige Verabreichung bei der Behandlung eines hormonabhängigen Zustands durch Modulation des Östrogenrezeptors β.

## Revendications

1. Utilisation d'un (i) anti-oestrogène et de (ii) trilostane ou de kétotrilostane dans la fabrication d'un médicament destiné au traitement d'un état pathologique hormono-dépendant par une modulation du récepteur des oestrogènes β, où le traitement comprend une administration simultanée dudit trilostane ou kétotrilostane et dudit anti-oestrogène.

2. Utilisation de trilostane ou de kétotrilostane dans la fabrication d'un médicament destiné à améliorer l'activité d'un anti-oestrogène utilisé pour le traitement d'un état pathologique hormono-dépendant, où le traitement comprend une administration simultanée dudit trilostane ou kétotrilostane et dudit anti-oestrogène.

3. Utilisation de trilostane ou de kétotrilostane dans la fabrication d'un médicament destiné à réduire les effets secondaires indésirables d'un anti-oestrogène utilisé pour le traitement d'un état pathologique hormono-dépendant.

4. Utilisation de trilostane ou de kétotrilostane dans la fabrication d'un médicament destiné à réduire ou à inverser le développement d'une résistance à un anti-oestrogène utilisé pour le traitement d'un état pathologique hormono-dépendant.

5. Utilisation de trilostane ou de kétotrilostane dans la fabrication d'un médicament destiné à être utilisé dans le traitement d'un état pathologique hormono-dépendant par une modulation du récepteur des oestrogènes β, par une administration simultanée avec un anti-oestrogène.

6. Utilisation d'un anti-oestrogène dans la fabrication d'un médicament destiné à être utilisé dans le traitement d'un état pathologique hormono-dépendant par une modulation du récepteur des oestrogènes β, par une administration simultanée avec du trilostane ou du kétotrilostane.

7. Utilisation selon l'une quelconque des revendications 1 à 6, dans lequel l'état pathologique hormono-dépendant est un état pathologique oestrogène-dépendant.

8. Utilisation selon l'une quelconque des revendications précédentes, dans laquelle l'état pathologique hormono-dépendant est un cancer hormono-dépendant.

9. Utilisation selon l'une quelconque des revendications précédentes, dans laquelle l'état pathologique hormono-dépendant est un cancer oestrogéno-dépendant.

10. Utilisation selon l'une quelconque des revendications précédentes, dans lequel l'état pathologique hormono-dépendant est un cancer du sein, un cancer de la prostate, un cancer du poumon, un cancer gastro-intestinal, un cancer de l'ovaire, un cancer de l'endomètre, une hypertrophie bénigne de la prostate, une hyperplasie de l'endomètre ou une hypertrophie de l'endomètre.

11. Utilisation selon l'une quelconque des revendications précédentes, dans laquelle le trilostane ou le kétotrilostane est sous une forme particulaire.

12. Utilisation selon la revendication 11, dans laquelle le trilostane ou le kétotrilostane est sous la forme de particules ayant un diamètre particulaire moyen compris entre 0 et 12 µm et 95 % des particules ou davantage ont un diamètre particulaire compris entre 0 et 50 µm.

13. Utilisation selon la revendication 12, dans laquelle les particules ont un diamètre particulaire moyen compris entre 5 et 12 µm.

14. Utilisation selon la revendication 12, dans laquelle les particules ont un diamètre particulaire moyen inférieur à 5 µm.

15. Utilisation selon l'une quelconque des revendications précédentes, dans laquelle l'unité posologique du trilostane ou du kétotrilostane est comprise entre 50 mg et 250 mg.

16. Utilisation selon l'une quelconque des revendications précédentes, dans laquelle l'anti-oestrogène est la tamoxifène ou le raloxifène.

17. Produit contenant (a) du trilostane ou du kétotrilostane et (b) un anti-oestrogène pour une utilisation combinée, par une administration simultanée, dans le traitement d'un état pathologique hormono-dépendant par une modulation du récepteur des oestrogènes β.
